# EUROPEAN PATENT SPECIFICATION

(11) **EP 2 628 499 B1**
(45) Date of publication and mention of the grant of the patent: **22.05.2019**
(21) Application number: 13168109.0
(22) Date of filing: 26.11.2008
(51) Int. Cl.: A61M 25/00, A61M 27/00

(54) **DRAINAGE CATHETER**
DRAINAGEKATHETER
CATHÉTER DRAINANT

(30) Priority: 29.11.2007 US 991097 P
(43) Date of publication of application: 21.08.2013
(62) Divisional of application: 08855183.3
(73) Proprietor: Boston Scientific Scimed, Inc., Maple Grove, MN 55311-1566 (US)
(72) Inventor: Chevalier, Raymond Jr., Elletsville, IN Indiana 47429 (US); Miller, Daniel, St. Louis, MO Missouri 63104 (US); Pickett, Steve, Spencer, IN Indiana 47460 (US); Wiese, Carla, Cincinnati, OH Ohio 45236 (US); Sherwood, Leslie, P., Elletsville, IN Indiana 47429 (US)
(74) Representative: Vossius & Partner Patentanwälte Rechtsanwälte mbB

(56) References cited:
- WO-A-2008/091652
- US-A- 5 205 830
- US-A- 5 472 435
- US-A- 5 489 269
- US-A1- 2002 091 352
- US-A1- 2003 153 873
- US-A1- 2006 258 982

## Description

### Background Information

Drainage catheters are often inserted percutaneously into abscesses or other structures to drain fluids. The shafts of these catheters are formed in a variety of sizes, durometers, and coatings. Holes located at the distal end of a typical drainage catheter allow fluids surrounding the catheter to enter a lumen through which they flow to the proximal end of the catheter out of the body. To enhance patient comfort, these catheters are generally made of soft biocompatible plastic. The soft material generally results in low column strength, so that a stiffening member is often inserted into a drainage catheter to allow it to be pushed into the body to a desired location. The stiffening member often abuts a surface near the distal end of the lumen at which the lumen tapers or steps down from a larger diameter, proximal portion to a reduced diameter, distal portion.

US 2002/0091352 A1 discloses a peritoneal dialysis catheter for insertion into the abdominal cavity of a patient for delivering and withdrawing fluid from a patient comprising a catheter body having a proximal portion, a distal tip portion and a first longitudinally extending lumen configured to deliver fluid into the abdominal cavity. A stiffening insert is portioned in the distal tip portion and has a first stiffness greater than a second stiffness of the distal tip portion to facilitate entry into the abdominal cavity. The stiffening insert has a lumen therethrough communicating with the first lumen.

US 2003/0153873 A1 shows a catheter comprising a soft flexible tube and a discrete segment of hard tubing that is press fit into the interior of the distal end of the soft tube.

Further, US 2006/0258982 A1 describes a catheter tip assembly comprising a marker band and a catheter tip. The catheter tip has an inner lumen therethrough and an outer portion and an inner portion with the marker band disposed therebetween. The outer tip portion and the inner tip portion can be made of different materials.

### Summary of the Invention

The present invention is directed to a catheter as defined in independent claim 1 and appended dependent claims.

### Brief Description of the Drawings

Figure 1 shows a drainage catheter assembly according to the invention;
Figure 2 shows a cross-sectional view of a catheter according to an example not forming part of the invention;
Figure 3 shows a cross-sectional view of a catheter according to an example not forming part of the invention;
Figure 4 shows a cross-sectional view of a catheter according to an embodiment of the present invention;
Figure 5 shows a cross-sectional view of a catheter according to another embodiment of the present invention; and
Figure 6 shows a cross-sectional view of a catheter according to an example not forming part of the invention.
Figure 7 shows a cross-sectional view of a catheter according to an example not forming part of the invention.
Figure 8 shows a cross-sectional view of a catheter according to an example not forming part of the invention.

### Detailed Description

The present invention may be further understood with reference to the following description and the appended drawings, wherein like elements are referred to with the same reference numerals. The invention relates to devices for draining fluid from the body. More specifically, the invention relates to a novel construction for a drainage catheter.

Drainage catheters typically are made of either a single material, or with a tip section separate from the catheter shaft. The separate tip section may be made of either a material with a different durometer than the catheter shaft, or a different material altogether.

As described above, stiffening members are often inserted into drainage catheters to aid in insertion. However, as the distal end of such a stiffening member is pressed into the catheter lumen, frictional drag along the outside of the catheter body may result in stretching of the catheter and/or the deformation of the material at the end of the lumen contacted by the stiffening member. In some cases, the stiffening member may become lodged within the tip section, preventing the removal of the stiffening member from the catheter. In such cases, the entire catheter must be removed and replaced, increasing discomfort and the time required for the procedure.

The catheter according to the present invention includes an abutting structure within the distal end of the lumen formed of a material having a higher durometer than that of the material of which the rest of the catheter is formed. The increased durometer of the abutting structure reduces the likelihood of the stiffening member becoming stuck within the tip section. The abutting structure is preferably formed of material of a higher modulus of elasticity than the material of which a shaft of the catheter is formed and may, more preferably be a dissolvable material as described in U.S. Patents 5,401,257 and 5,049,138.

The abutting structure according to the embodiments of this invention may be fused to the catheter shaft material within the tip section as part of the tipping process by, for example, mechanical bonding (e.g., press fit, or snap fit) and/or chemical bonding (e.g., heat fusion, adhesives, or solvents). Alternatively, the abutting structure may be added to the catheter shaft material in a process other than the tipping process. These alternative processes may include a separate adhesive process, a solvent bonding process, a mechanical fit process, or an over-molding process. An outer diameter of the abutting structure preferably mates with an inner diameter of a portion of the catheter shaft within which it is to reside while an inner diameter of the abutting structure is preferably substantially a desired inner diameter of a portion of the lumen extending through the tip section so that the abutting structure does not interfere with fluid flow through the lumen.

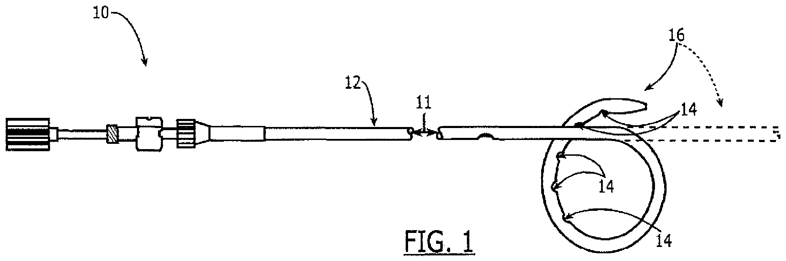

As shown in Fig. 1, a drainage catheter assembly 10 according to the present invention includes a lumen 11 extending through a shaft 12 from the distal end to the proximal end thereof. Generally, multiple holes 14 near the distal end of the catheter shaft 12 allow fluids to drain out of the body. As would be understood by those skilled in the art, in order to assist in maintaining the catheter at a desired location (e.g., within a chamber of a lumen) the tip section 16 of the catheter shaft 12 may have a curled, 'pigtail' shape which anchors the drainage catheter assembly 10 at the outlet from the organ chamber to a lumen through which the catheter assembly 10 was introduced thereinto.

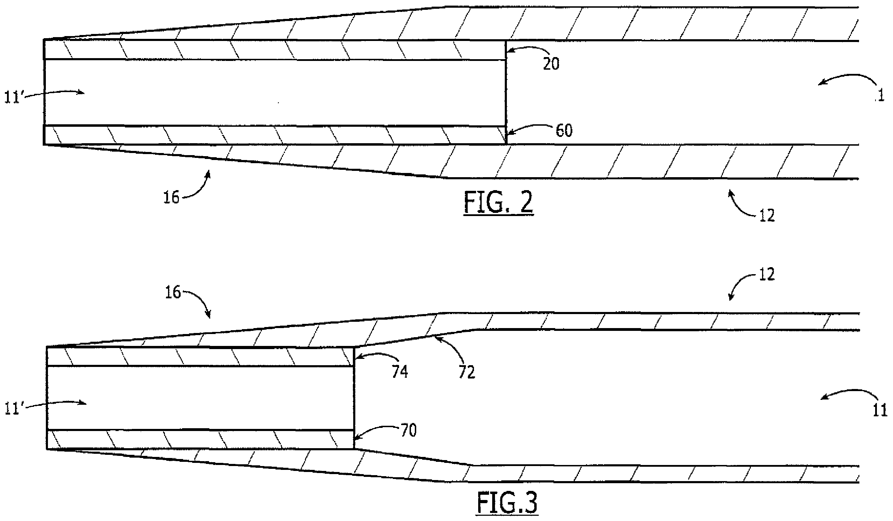

As shown in Fig. 2, tip 16 including an abutting structure 60 forms a stepped transition 20 in the ,lumen 11. The abutting structure 60 is formed as a ring mounted within the lumen 11 with the abutting structure 60 being formed of a material stiffer than the material of which the rest of the catheter 10 is formed. An outer diameter of the abutting structure 60 is substantially equal to an inner diameter of the lumen 11 to mate with the inner surface of the catheter shaft 12. The abutting structure 60 extends through the entire tip section 16 to end at the distal opening of the catheter 10. The inner diameter of the abutting structure 60 defines a narrower inner diameter lumen portion 11' of the lumen 11 extending through the tip section 16. Furthermore, a proximal end of the abutting structure 60 defines an increased stiffness stepped transition 20' within the lumen portion 11'. Thus, when a stiffening member is pushed against the stepped transition 20, a material of the stepped transition 20 is less likely to deform, thus reducing the likelihood of the stiffening member becoming lodged within the tip section 16, as those skilled in the art will understand. The catheter 10 and, the abutting structure 60 may be formed of one of the same material and different materials. The materials for either one may include, for example, any type of thermoplastic polyurethane or low density polyurethane, silicone, ethylene vinyl acetate (EVA), or polyvinyl chloride (PVC), etc. The materials may also include a filler such as, for example, a radiopacifier or· anti-microbial agent. The abutting structure may each have a different hardness depending on the material chosen. If a material of the abutting structure 60 is the same as that of the catheter 10, it may only need to be of higher durometer than that of the shaft 12 in order to function as indicated above. Alternatively, the abutting surface may be formed of high density pollethylene (HDPE), nylon, polycarbonate, acrylonitrile butadiene styrene (ABS) and aluminum. Catheters made 6fthermoplastic polyurethane are commonly found in the durometer range of Shore 72A -Shore 84D, as would be understood by those skilled in the art.

As shown in Fig. 3, a tip 16 including an abutting structure 70 is mounted distally of a tapered tip section 72 of the lumen 11. The abutting structure 70 is formed as a ring of a material stiffer than that of which the rest of the catheter 10 is formed and is mated within the reduced diameter lumen portion 11'. The tapered tip section 72 reduces the diameter of lumen 11 in a distal direction and the abutting structure 70 forms a further stepped reduction in this diameter. The abutting structure 70 extends through the tip 16 from the distal end of the tapered tip section 72 to the distal end of the catheter 10. As in the previous examples, the outer diameter of the abutting structure 70 is substantially equal to the inner diameter of the lumen portion 11' (i. e., less than this inner diameter of the distal portion of the lumen 11 by only a tolerance amount necessary to facilitate insertion therein). The portion of the lumen 11 extending through the abutting structure 70 may have a substantially constant diameter along its length. Alternatively, the lumen portion 11' extending through the abutting structure 70 may comprise a tapered diameter. In another alternate example, the tapered tip section 72 may extend along all or apart of the length of the abutting structure 70 with a corresponding taper on an outer surface of the abutting structure 70. As described above, the abutting structure 70 increases the stiffness of the tapered tip section 72 to reduce the likelihood that of a stiffening member being lodged within the tip section 16 or plastically deforming the tip section 16.

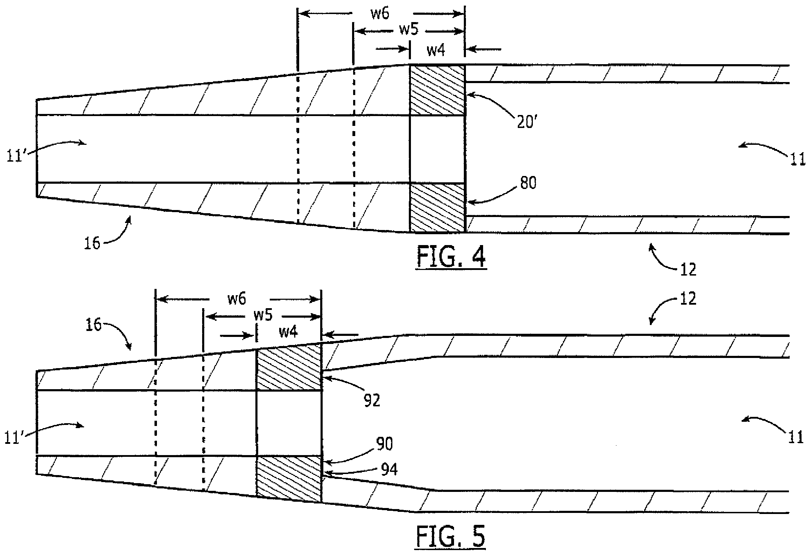

As shown in Fig.4, an abutting structure 80 according to an embodiment of the invention is formed at a proximal end of the tip 16. The abutting structure 80 is formed as a ring of higher stiffness material at a point where the catheter shaft 12 is joined to the, tip section 16. The abutting structure 80 may be formed with any desired width w (e.g., widths w4, w5, or w6), depending on the requirement of a procedure to be performed therewith. The outer diameter of the proximal end of the abutting structure 80 matches the outer diameter of the distal end of the catheter shaft 12 to form a continuous, smooth transition from the catheter shaft 12 to the tip 16. Similarly, the outer diameter of the distal end of the abutting structure 80 matches the outer diameter of the proximal end of the tip section 16, to·also form a smooth transition therewith. The inner diameter of the abutting structure 80 is approximately equal to an inner diameter of the lumen portion 11' extending through the tip section 16. In this manner, the proximal end of the abutting surface 80 forms an increased stiffness stepped transition 20' to the lumen portion 11'. Thus, as a stiffening member pushes on the stepped transition 20' during catheter insertion, the abutting surface 80 reduces the likelihood that the stiffening member will become lodged within the tip section 16.

As shown in Fig. 5, an abutting structure 90 according to another embodiment of the invention is formed at a proximal end of the tip 16. The abutting structure 90 is formed of a relatively stiff material in the shape of a ring joining a tapered transition 30 of the catheter shaft 12 to the tip section 16. Similar to the embodiment of Fig. 4 described above, the abutting structure 90 may have any desired width w (e.g., widths w4, wS, w6, etc.) depending on the desired characteristics of the tip 16. The outer diameter of a proximal end of the abutting structure 90 is substantially equal to that of the distal end of the tapered transition 30 of the catheter body 12 to form a smooth transition therebetween. However, those skilled in the art will understand that any profile of each of the distal end of the catheter body 12, the abutting structure 90 and the tip 16 may be selected to achieve a desired overall contour of the distal end of the catheter 10 so long as transitions between these parts are smooth. The inner diameter of the abutting structure 90 is approximately equal to an inner diameter of the lumen portion 11' extending through the tip section 16. Thus, a proximal face 92 of the abutting structure 90 forms a stepped transition 94 at an interface with the shaft 12. As in the preceding embodiments, since the abutting structure 90 is formed of a stiffer material than the rest of the catheter 10, the stiffness of the stepped transition 94 is also increased. Thus, as a stiffening member pushes on the stepped transition 94 during catheter insertion, the proximal face 92 of the abutting structure 90 reduces the likelihood that the stiffening member will become lodged within the tip section 16.

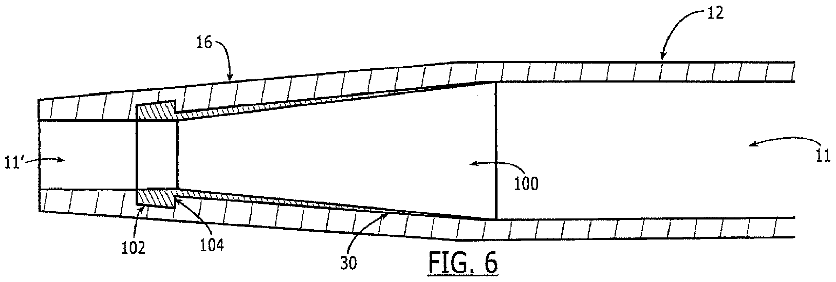

As shown in Fig. 6, an abutting structure 100 is formed as a liner of shell within the lumen portion 11' of the tip 16. The abutting structure 100 is formed of a material of increased stiffness relative to the material of which the catheter 10 is formed and extends along a tapered transition 30 of the tip 16. An outer diameter of the abutting structure 100 mates with an inner diameter of the tapered transition 30 with a tapered proximal thickness to create a smooth inner surface transition from the lumen 11 to the lumen portion 11', as those skilled in the art will understand. The abutting structure 100 further comprises a locating feature 102 which locks in a mating recess 104 formed within an inner diameter of the tip section 16 adjacent a distal end of the tapered transition 30. Thus, when the locating feature 102 is seated within the mating recess 104, the abutting structure 100 assumes a locked configuration relative to the tip section 16, thereby increasing the stiffness of the tapered transition 30 and forming a hard shell around the tapered portion of the lumen portion 11'.

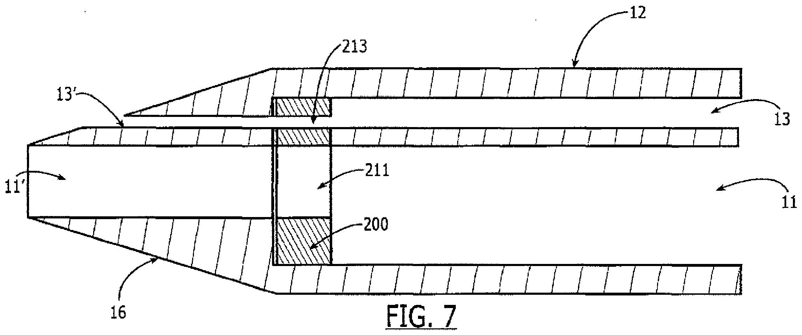

Fig. 7 shows an abutting structure 200 formed at a proximal end of the tip 16 of a shaft 12 of a multilumen catheter. The abutting structure 200 is also formed as a ring of a higher stiffness material relative to the catheter shaft 12 and is situated adjacent a joint between the catheter shaft 12 and the tip 16. Specifically, the abutting structure 200 extends across the distal end of the lumen 11, past a partition dividing the lumen 11 from a second lumen 13 and abuts a portion of the wall of the lumen 13 formed by an outer wall of the shaft 12. In this embodiment, the abutting structure 200 is located proximally of the tapered tip section 16. The abutting structure 200 further comprises a first lumen 211 extending therethrough and substantially aligning with the lumen 11 so that, when the abutting structure is positioned as noted above, the lumen 211 connects the lumen 11 to the lumen 11'. A lumen 213 extending through the abutting structure 200 further aligns with the lumen 13, connecting the lumen 13 to a lumen 13' extending through the tip 16. Those skilled in the art will understand that the lumens 13, 213 and 13' open to the distal end of the tip 16 and may be used, for example, to provide aspiration, supply fluids, etc. The lumens 11, 211 and 11' may then be used for drainage as described above.

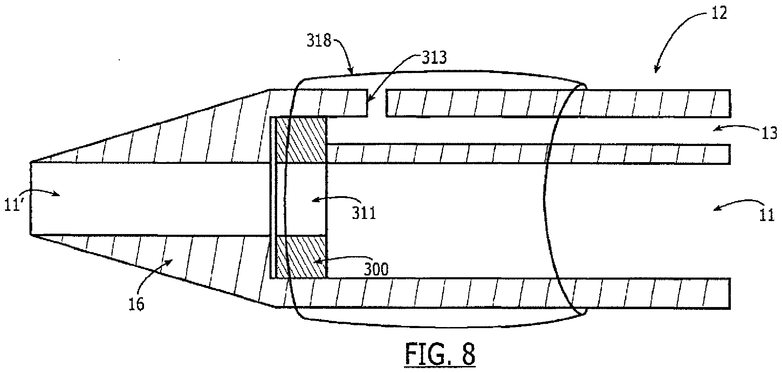

Fig. 8 shows an abutting structure 300 wherein the abutting structure 300 is formed as a ring at a distal end of the catheter shaft 12. Similar to the previously described examples, the abutting structure 300 is formed as a ring of a higher stiffness material located between the catheter shaft 12 and the tip 16. The abutting structure 300 extends across the distal end of the inner diameter of the lumen 11, through a partition dividing the lumen 11 from the lumen 13 to abut a portion of the wall of the lumen 13 formed by an outer wall of the shaft 12. In this embodiment, the abutting structure 300 is located proximally of the tapered tip section 16. The abutting structure 300 comprises a lumen 311 extending therethrough substantially aligning with the lumen 11 to connect the lumen 11 to the lumen 11' of the tip 16, thus providing a stepped diameter from the lumen 11 to the lumen 11'. A portion of the abutting structure 300 extends across and closes a distal end of the lumen 13. The catheter shaft 12 further comprises an inflation port 313 connecting the lumen 13 to an exterior of the shaft 12 which is enveloped in a balloon 318. Thus, the provision of inflation fluid to the lumen 13 inflates the balloon 318 to, for example, abut adjacent tissue and lock the catheter in position.

The abutting structures of the catheters according to the embodiments of the present invention and examples are shown in the appended figures as rings which substantially match the internal shape of the lumen of the catheter. However, alternative designs other than rings (e.g., projections into the lumen) may also be used so long as they provide a stiffer surface for engaging a stiffening member inserted into the catheter. These shapes preferably conform to the shape of the lumen of a single lumen catheter. However, those skilled in the art will understand that for multiple lumen catheters, the shape of an abutting surface may conform to a shape of an outer wall of the catheter, to the shapes of the lumens themselves or may simply form projections into the lumens against which the stiffening member(s) will push. Additionally, the abutting structures may be a variety of sizes (e.g., widths, thicknesses, or diameters) for use in a variety of catheters, for extra stiffening capacity, or for ease of manufacture. Further, one or more abutting structures may be added to multi-lumen drainage catheters as well.

The concept of adding a stiffer material to a catheter shaft may also be used for other products (e.g., venous access catheters such as dialysis, tunneled central, and PICC (peripherally inserted central catheters)) that have flexible shafts into which an insertion tool or other device is inserted to impart a force to the product through contact with an internal structure which might be damaged if not formed of a material more stiff than that of which the rest of the catheter is formed. For example, such a construction may be employed for pacemaker leads, aneurism coils, removable core guidewires, introducer sheaths for vascular access, etc. Further, the secondary material may be added along the entire length of the shaft or only along any portion of the shaft as desired, such as the tip section as shown in the exemplary embodiments.

The present invention has been described with reference to specific embodiments, and more specifically to an abutting structure for use with drainage catheters. However, other embodiments may be devised that are applicable to other medical devices and procedures, without departing from the scope of the invention. Accordingly, various modifications and changes may be made to the embodiments, without departing from the broadest scope of the present invention as set forth in the claims that follow. The specification and drawings are accordingly to be regarded in an illustrative rather than restrictive sense.

## Claims

1. A catheter (10) including:
a shaft (12) formed of a first material, the shaft (12) defining a shaft lumen (11) extending therethrough;
a distal tip (16) including a tip lumen (11') extending therethrough, and
a stiffening member,
**characterized in that**
the stiffening member is an abutting member (80, 90) formed of a second material stiffer than the first material, the abutting member (80, 90) defining an abutting member lumen (11') extending therethrough, a proximal end of the abutting member (80, 90) coupled to a distal end of the shaft (12), a proximal surface of the abutting member (80, 90) forming an abutting surface at least partially covering the shaft lumen (11), an outer diameter of the proximal end of the abutting member (80, 90) matching an outer diameter of the distal end of the shaft (12); and a proximal end of the distal tip (16) coupled to a distal end of the abutting member (80, 90), an outer diameter of the proximal end of the distal tip (16) matching the outer diameter of the distal end of the abutting member (80, 90), an inner diameter of the abutting member lumen (11') matching an inner diameter of the proximal end of the distal tip (16).

2. The catheter of claim, 1, wherein the inner diameter of the abutting member (80, 90) is smaller than an inner diameter of the shaft (12).

3. The catheter of claim 1, wherein the distal tip (16) is formed of the first material.

4. The catheter of claim 1, wherein the distal tip (16) is formed of a third material, the first material being stiffer than the third material.

5. The catheter of claim 1, wherein the inner diameter of the abutting member (80, 90) is constant along its length.

6. The catheter of claim 1, wherein the inner diameter of the distal tip (16) extends through a length of the distal tip (16).

7. The catheter of claim 1, wherein the outer diameter of the distal tip (16) tapers from the proximal end to a distal end thereof.

8. The catheter of claim 1, wherein the outer diameter of the abutting member (80, 90) is the same as the outer diameter of the shaft (12) forming a continuous, smooth transition therebetween, and wherein the outer diameter of the proximal end of the distal tip (16) is the same as the outer diameter of the shaft (12).

9. The catheter of claim 1, wherein the abutting member (80, 90) includes a proximal portion having an outer diameter that is the same as the outer diameter of the shaft (12) along a length thereof and a distal portion having an outer diameter that tapers to the distal end of the abutting member (80, 90).

10. The catheter of claim 9, wherein the distal portion of the abutting member (80, 90) tapers to form a continuous, smooth transition with a tapering of the distal tip (16).

11. The catheter of claim 1, wherein the outer diameter of a distal portion of the shaft (12) tapers toward a distal end thereof.

12. The catheter of claim 11, wherein the outer diameter of the abutting member (80, 90) tapers from the proximal end to the distal end thereof.

13. The catheter of claim 12, wherein the abutting member (80, 90) tapers to form a continuous, smooth transition with a tapering of the distal tip (16).

## Patentansprüche

1. Katheter (10), der aufweist:
einen Schaft (12), der aus einem ersten Material gebildet ist, wobei der Schaft (12) ein sich dadurch erstreckendes Schaftlumen (11) definiert;
eine distale Spitze (16) mit einem sich dadurch erstreckenden Spitzenlumen (11') und einem Versteifungsteil,
**dadurch gekennzeichnet, dass**
das Versteifungsteil ein Anlageteil (80, 90) ist, das aus einem zweiten Material gebildet ist, das steifer als das erste Material ist, wobei das Anlageteil (80, 90) ein sich dadurch erstreckendes Anlageteillumen (11') definiert, ein proximales Ende des Anlageteils (80, 90) mit einem distalen Ende des Schafts (12) gekoppelt ist, eine proximale Fläche des Anlageteils (80, 90) eine Anlagefläche bildet, die mindestens teilweise das Schaftlumen (11) abdeckt, ein Außendurchmesser des proximalen Endes des Anlageteils (80, 90) einem Außendurchmesser des distalen Endes des Schafts (12) entspricht; und ein proximales Ende der distalen Spitze (16) mit einem distalen Ende des Anlageteils (80, 90) gekoppelt ist, ein Außendurchmesser des proximalen Endes der distalen Spitze (16) dem Außendurchmesser des distalen Endes des Anlageteils (80, 90) entspricht und ein Innendurchmesser des Anlageteillumens (11') einem Innendurchmesser des proximalen Endes der distalen Spitze (16) entspricht.

2. Katheter nach Anspruch 1, wobei der Innendurchmesser des Anlageteils (80, 90) kleiner als ein Innendurchmesser des Schafts (12) ist.

3. Katheter nach Anspruch 1, wobei die distale Spitze (16) aus dem ersten Material gebildet ist.

4. Katheter nach Anspruch 1, wobei die distale Spitze (16) aus einem dritten Material gebildet ist, wobei das erste Material steifer als das dritte Material ist.

5. Katheter nach Anspruch 1, wobei der Innendurchmesser des Anlageteils (80, 90) über dessen Länge konstant ist.

6. Katheter nach Anspruch 1, wobei sich der Innendurchmesser der distalen Spitze (16) durch eine Länge der distalen Spitze (16) erstreckt.

7. Katheter nach Anspruch 1, wobei sich der Außendurchmesser der distalen Spitze (16) vom proximalen Ende zu einem distalen Ende davon verjüngt.

8. Katheter nach Anspruch 1, wobei der Außendurchmesser des Anlageteils (80, 90) gleich dem Außendurchmesser des Schafts (12) ist, was einen kontinuierlichen, glatten Übergang dazwischen bildet, und wobei der Außendurchmesser des proximalen Endes der distalen Spitze (16) gleich dem Außendurchmesser des Schafts (12) ist.

9. Katheter nach Anspruch 1, wobei das Anlageteil (80, 90) aufweist: einen proximalen Abschnitt mit einem Außendurchmesser, der gleich dem Außendurchmesser des Schafts (12) über eine Länge davon ist, und einen distalen Abschnitt mit einem Außendurchmesser, der sich zum distalen Ende des Anlageteils (80, 90) verjüngt.

10. Katheter nach Anspruch 9, wobei sich der distale Abschnitt des Anlageteils (80, 90) verjüngt, um einen kontinuierlichen, glatten Übergang mit einer Verjüngung der distalen Spitze (16) zu bilden.

11. Katheter nach Anspruch 1, wobei sich der Außendurchmesser eines distalen Abschnitts des Schafts (12) zu einem distalen Ende davon verjüngt.

12. Katheter nach Anspruch 11, wobei sich der Außendurchmesser des Anlageteils (80, 90) vom proximalen Ende zum distalen Ende davon verjüngt.

13. Katheter nach Anspruch 12, wobei sich das Anlageteil (80, 90) verjüngt, um einen kontinuierlichen, glatten Übergang mit einer Verjüngung der distalen Spitze (16) zu bilden.

## Revendications

1. Cathéter (10) incluant :
une tige (12) formée d'un premier matériau, la tige (12) définissant une lumière de tige (11) s'étendant dans celle-ci ;
un bout distal (16) incluant une lumière de bout (11') s'étendant dans celui-ci, et
un élément rigidifiant
**caractérisé en ce que**
l'élément rigidifiant est un élément d'aboutement (80, 90) formé d'un second matériau plus rigide que le premier matériau, l'élément d'aboutement (80, 90) définissant une lumière d'élément d'aboutement (11') s'étendant dans celui-ci, une extrémité proximale de l'élément d'aboutement (80, 90) couplée à une extrémité distale de la tige (12), une surface proximale de l'élément d'aboutement (80, 90) formant une surface d'aboutement couvrant au moins en partie la lumière de tige (11), un diamètre externe de l'extrémité proximale de l'élément d'aboutement (80, 90) s'adaptant à un diamètre externe de l'extrémité distale de la tige (12) ; et une extrémité proximale du bout distal (16) couplée à une extrémité distale de l'élément d'aboutement (80, 90), un diamètre externe de l'extrémité proximale du bout distal (16) s'adaptant au diamètre externe de l'extrémité distale de l'élément d'aboutement (80, 90), un diamètre interne de la lumière d'élément d'aboutement (11') s'adaptant à un diamètre interne de l'extrémité proximale du bout distal (16).

2. Cathéter selon la revendication 1, où le diamètre interne de l'élément d'aboutement (80, 90) est plus petit qu'un diamètre interne de la tige (12).

3. Cathéter selon la revendication 1, où le bout distal (16) est formé du premier matériau.

4. Cathéter selon la revendication 1, où le bout distal (16) est formé d'un troisième matériau, le premier matériau étant plus rigide que le troisième matériau.

5. Cathéter selon la revendication 1, où le diamètre interne de l'élément d'aboutement (80, 90) est constant le long de sa longueur.

6. Cathéter selon la revendication 1, où le diamètre interne du bout distal (16) s'étend sur une longueur du bout distal (16).

7. Cathéter selon la revendication 1, où le diamètre externe du bout distal (16) va en se rétrécissant de l'extrémité proximale à une extrémité distale de celui-ci.

8. Cathéter selon la revendication 1, où le diamètre externe de l'élément d'aboutement (80, 90) est le même que le diamètre externe de la tige (12) formant une transition uniforme continue entre eux, et où le diamètre externe de l'extrémité proximale du bout distal (16) est le même que le diamètre externe de la tige (12).

9. Cathéter selon la revendication 1, où l'élément d'aboutement (80, 90) inclut une partie proximale ayant un diamètre externe qui est le même que le diamètre externe de la tige (12) le long d'une longueur de celle-ci et une partie distale ayant un diamètre externe qui va en se rétrécissant jusqu'à l'extrémité distale de l'élément d'aboutement (80, 90).

10. Cathéter selon la revendication 9, où la partie distale de l'élément d'aboutement (80, 90) va en se rétrécissant pour former une transition uniforme continue avec un rétrécissement du bout distal (16).

11. Cathéter selon la revendication 1, où le diamètre externe d'une partie distale de la tige (12) va en se rétrécissant en direction d'une extrémité distale de celle-ci.

12. Cathéter selon la revendication 11, où le diamètre externe de l'élément d'aboutement (80, 90) va en se rétrécissant de l'extrémité proximale à l'extrémité distale de celui-ci.

13. Cathéter selon la revendication 12, où l'élément d'aboutement (80, 90) va en se rétrécissant pour former une transition uniforme continue avec un rétrécissement du bout distal (16).
